# EUROPEAN PATENT SPECIFICATION

(11) **EP 3 749 382 B1**
(45) Date of publication and mention of the grant of the patent: **17.04.2024**
(21) Application number: 19704269.0
(22) Date of filing: 04.02.2019
(51) Int. Cl.: A61N 1/36, A61M 60/191, A61M 60/289, A61M 60/468, A61M 60/515, A61M 60/839

(54) **DETERMINING CONTROL PARAMETERS FOR CARDIAC AUGMENTATION DEVICES**
BESTIMMUNG VON STEUERPARAMETERN FÜR KARDIALE AUGMENTATIONSGERÄTE
DÉTERMINATION DE PARAMÈTRES DE COMMANDE POUR DISPOSITIFS D'AUGMENTATION CARDIAQUE

(30) Priority: 06.02.2018 DE 102018201825
(43) Date of publication of application: 16.12.2020
(73) Proprietor: AdjuCor GmbH, 81673 München (DE)
(72) Inventor: MAIER, Andreas, 85567 Grafing b. München (DE); WILDHIRT, Stephen, Manuel, 82335 Berg (DE); CLAUSS, Johannes Friedrich, 82234 Weßling (DE); HOCHREITER, Johannes, 86157 Augsburg (DE); GRAF FINCK VON FINCKENSTEIN, Roland, 81735 München (DE)
(74) Representative: Peterreins Schley
(86) International application number: PCT/EP2019/052666
(87) International publication number: WO 2019/154764

(56) References cited:
- EP-B1- 1 331 970
- US-A1- 2006 069 322
- US-A1- 2008 119 903
- US-B1- 6 567 700

## Description

### Technical Field

This disclosure relates to cardiac augmentation devices, computer program products for cardiac augmentation devices and methods for determining control parameters for cardiac augmentation devices.

### Background

As a result of one or more of numerous pathological conditions, the pumping capability of a heart can be impaired. In many cases, the heart can be unable to pump sufficient blood to maintain an appropriate blood flow. This state is also referred to as heart failure or congestive heart failure (CHS). In the recent years, the number of patients suffering from congestive heart failure has continuously increased. In the United States alone, approximately 5 million people suffer under congestive heart failure. For Europe or other developed countries, similar numbers apply.

One reason for the occurrence of a congestive heart failure can be an impaired contraction ability of the heart due to a damage of the heart muscle. In other examples, the pumping function of the heart can be impaired by leaky valve, for example a leaky aortic valve or a leaky mitral valve. In still other examples, arrhythmias can cause an impaired pumping function of the heart. This list can be continued.

Different types of chronic heart failure can be treated by surgery or pharmaceutically. For instance, a defective heart valve can be replaced surgically, or an arrhythmia can be treated by an implantable medical device. In still other examples, a pumping device can be implanted to replace or support the pumping function of the heart. The latter approach is also called cardiac augmentation. For example, one or more actuators can be controlled in synchronicity with the natural heartbeat of the heart to augment the impaired pumping function of the heart. Controlling the operation of cardiac augmentation devices is not straightforward.

US 2008/119903 A1 and US 2006/069322 A1 disclose systems to provide pacing to a heart to improve pumping efficiency of the heart using cardiac activation sequence information.

### Summary

The invention is defined by the appended claims.

In one general aspect, the present disclosure relates to a cardiac augmentation device including an actuator configured to apply pressure to a heart to augment a pumping function of the heart, a sensor system configured to record two or more different ECG leads at the augmented heart. The cardiac augmentation device additionally includes a controller configured to determine one or more control parameters for the cardiac augmentation device using one or more of the two or more ECG leads and control operation of the actuator based on the determined one or more control parameters.

In a second general aspect, the present disclosure relates to a computer program product including instructions stored thereon which when executed by a controller of a cardiac augmentation device prompt the cardiac augmentation device to record two or more different ECG leads at an augmented heart, determine one or more control parameters for the cardiac augmentation device using one or more of the two or more ECG leads of the cardiac augmentation device and control operation of an actuator of the cardiac augmentation device based on the determined one or more control parameters.

In a third general aspect, the present disclosure relates to a method for determining control parameters for a cardiac augmentation device, the cardiac augmentation device including an actuator configured to apply pressure to a heart to augment a pumping function of the heart, the method including recording two or more different ECG leads at an augmented heart and determining one or more control parameters for the cardiac augmentation device using one or more of the two or more ECG leads.

The techniques according to the first to third general aspects can have one or more of the following advantages in some examples.

Firstly, augmentation parameters can be determined more reliably in some examples when using the techniques of the present disclosure. For instance the technique of the present disclosure can involve using a plurality of ECG leads to determine an augmentation start or an augmentation end point (or any other augmentation control parameter). The controller of the cardiac augmentation device can select one or more of the plurality of ECG leads to determine the augmentation control parameter. In this manner, a more robust determination of the respective augmentation control parameter can be achieved. If an augmentation parameter is only determined based on a single sensor channel (e.g., ECG lead), errors occurring on this single channel might render the determined cardiac augmentation parameter useless.

For instance, an electrode element of a single channel sensor element can become detached or pick up noise which can make determining the augmentation control parameters difficult. When using the techniques of the present disclosure, a controller might switch to another channel and still reliably determine the augmentation control parameters.

Secondly, when using the techniques of the present disclosure a latency of the controller determining the augmentation control parameter can be relatively low. In some examples, the augmentation parameters can be determined substantially in real time (i.e., characteristics of the signals of the plurality of ECG leads can be used to determine augmentation control parameters for the very same heartbeat). In other examples, the latency can be even lower (e.g., less than 2 ms or less than 1 ms in some examples).

Thirdly, using information from two or more ECG leads can facilitate deriving additional information regarding pathological or non-pathological conditions of the augmented heart. This can be helpful to further improve the determination of the augmentation control parameters and therefore the augmentation outcome. For example, an augmented heart might suffer from different pathological (or non-pathological) conditions. This information can be used to adapt the augmentation process itself or auxiliary functions of a cardiac augmentation device.

Several terms are used in the present disclosure in a particular manner.

In the present disclosure, the term "cardiac augmentation" is used to indicate that a contracting function of the heart is augmented by a device. In other words, the device does not completely replace the function of the heart (which is the case when using some systems that include a continuously operating pump or other similar devices). Rather, cardiac augmentation involves operating synchronously with a naturally occurring pumping function of the heart to increase the output of the heart.

Accordingly, a "cardiac augmentation device" is a device configured to perform cardiac augmentation. In some passages of the present disclosure a particular type of cardiac augmentation device (e.g., including a plurality of inflatable and deflatable pads) is used as exemplary device to explain aspects of the present disclosure. However, the techniques of the present disclosure are not limited to this particular type of device. In general, the techniques of the present disclosure can be used in any cardiac augmentation device having an actuator configured to apply pressure to a heart to augment a pumping function of the heart in synchronicity with a naturally occurring heartbeat.

The term "heartbeat" is used in the present disclosure to label a period in time in which a single contraction (and relaxation) of the heart takes place.

The attributes "naturally occurring" and "natural" in the context of a heartbeat in the present disclosure also include heartbeats induced by a rhythm management device (e.g., a pacemaker). Again, the attributes "naturally occurring" and "natural" shall convey that the techniques of the present disclosure relate to augmentation devices which augment a natural pumping function of the heart.

The term "lead" in the present disclosure defines a particular measurement vector of electrical activity of the heart. The orientation of the measurement vector of a lead is substantially fixed (e.g., between a first electrode applied epicardially at the heart and a reference electrode, or between two electrodes attached to the heart). A "lead" does not directly correspond to a piece of hardware (e.g., a set of dedicated electrodes or a connector to a set of electrodes). In some examples there is dedicated hardware to record a particular lead (e.g., a set of two electrodes arranged in a particular spatial relationship).

However, in other examples a lead can be calculated from signals sensed by multiple electrodes. For instance, if three electrodes are arranged at a heart, a sensor system can be configured to record signals indicating electrical activity of the heart for each of the three electrodes compared to a reference electrode (e.g., a can of an implanted device). Each of the three recorded signals is the signal of one lead in this example. Based on the recorded signals, additional leads can be calculated. For instance, a lead between any pair of the three electrodes can be calculated which also is the signal of a different lead. In another example, a combined signal of the three electrodes compared to the reference electrode can be an additional lead of the sensor system. In examples with more than three electrodes, other and additional combinations of signals to calculate leads are possible. In other words, electrodes and their connectors provided by the sensor system provide the hardware which than can be used to record the two or more different ECG leads according to the present disclosure by appropriately processing measurement signals of the electrodes. As a result of the aforesaid, whenever a lead is "recorded" according to the present disclosure, this can include a (direct) sensing of the respective lead between two electrodes or a calculation of the respective lead using one or more measurement signals of the sensing system.

The terms "control parameter" and "augmentation control parameter" include any parameter which can be used to control operation of an actuator of a cardiac augmentation device.

The term "controller" in the present disclosure relates to an entity that is adapted to carry out a set of operations. The controller is not limited in view of its hardware in other way than by being adapted to carry out these operations. In particular, a controller might not be embodied in a single component but can also be distributed over different components (which might even be located remote from a patient carrying the cardiac augmentation device).

### Description of the Drawings

**FIGS. 1****,** **2A and 2B** illustrate an example cardiac augmentation device according to the present disclosure.
**FIG. 3** shows example pressure, volume and ECG signals of a heart.
**FIG. 4A** depicts an example signal of an ECG lead and corresponding pressure curves of an example cardiac augmentation device according to the present disclosure.
**FIG. 4B** depicts example signals of a plurality of ECG leads according to the present disclosure.
**FIG. 5** includes a flow diagram of an example method according to the present disclosure.

### Detailed Description

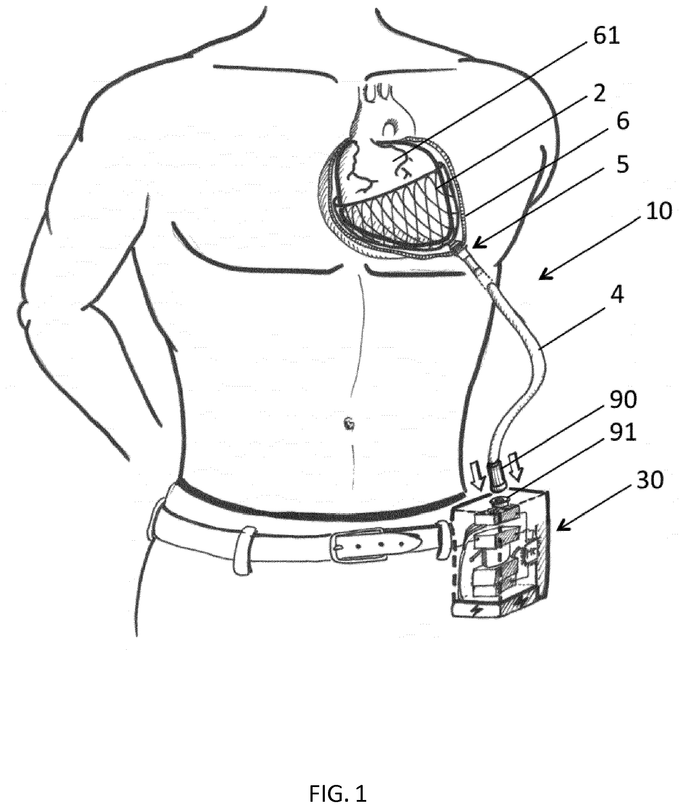

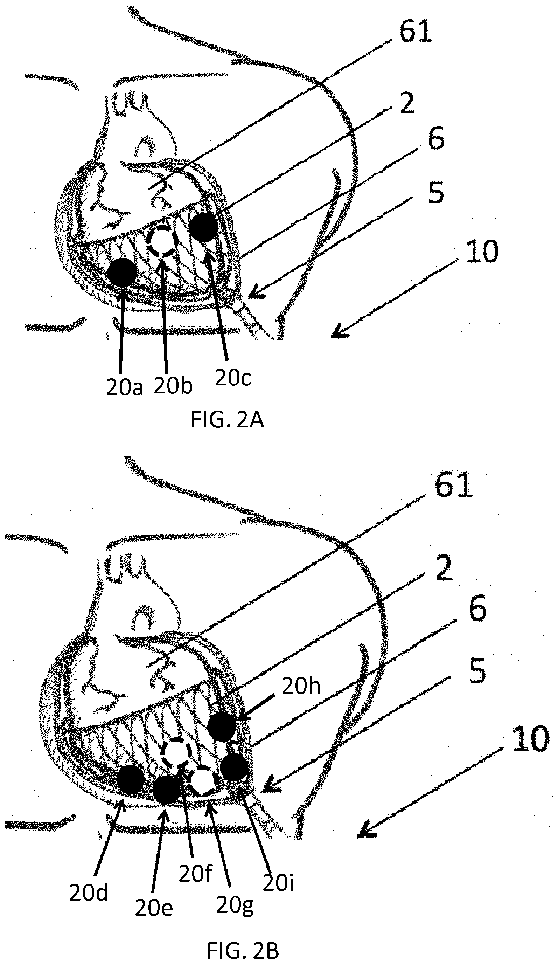

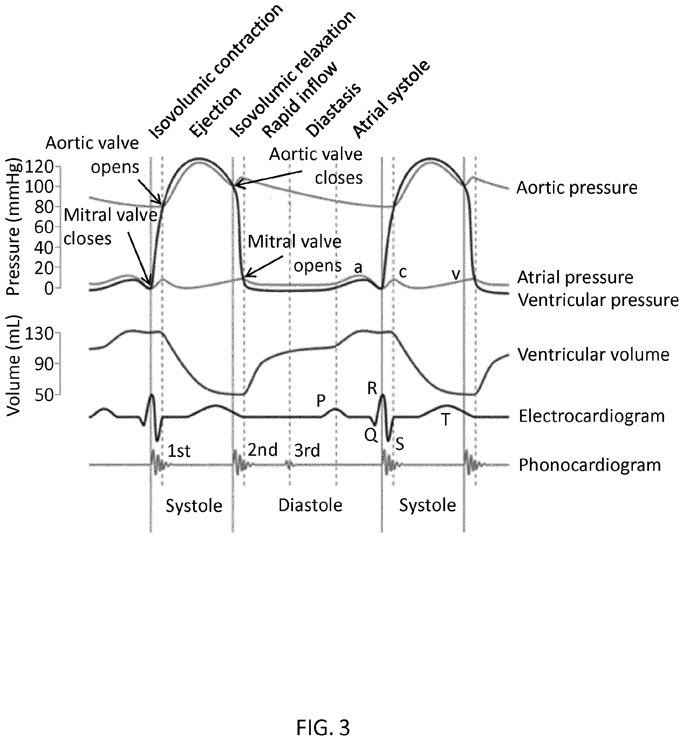

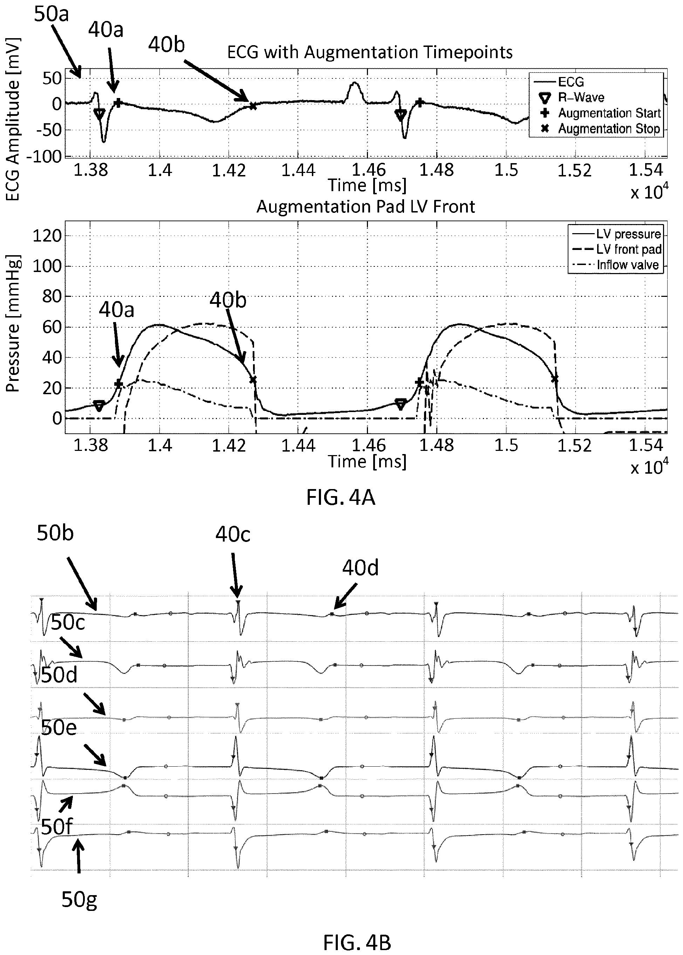

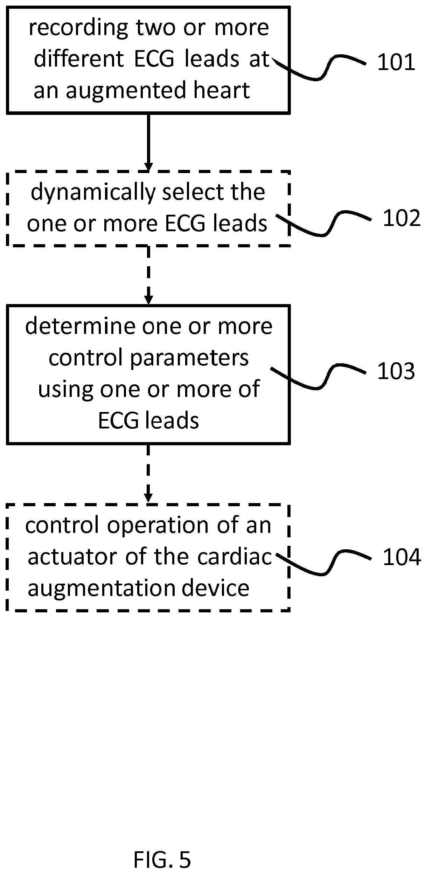

The cardiac augmentation devices, computer program products for cardiac augmentation devices and methods for determining control parameters for cardiac augmentation devices according to the present disclosure will be discussed in more detail in the subsequent sections. First, an example cardiac augmentation device in which the techniques of the present disclosure can be employed will be discussed in connection with **FIGS. 1****,** **2A** and **2B****.** Next, several basic aspects of the relationship of different physiological parameters of the heart which are helpful to understand aspects of the present disclosure will be explained in connection with **FIG. 3****.** Subsequently, different aspects of the determination of control parameters according to the present disclosure will be discussed in connection with **FIGS. 4A, 4B****, and** **5****.**

### Hardware for cardiac augmentation device

**FIG. 1** shows an example cardiac augmentation device 10 according to the present disclosure. The cardiac augmentation device in **FIG. 1** serves only for the sake of illustration. As discussed above, the techniques of the present disclosure generally are not specific to a particular augmentation device (as long as it has an actuator configured to apply pressure to a heart and a sensor system configured to sense two or more different ECG leads).

Coming back to **FIG. 1****,** the cardiac augmentation device 10 includes an actuator 2 configured to apply pressure to a heart 61 to augment a pumping function of the heart and a sensor system (not shown in **FIG. 1****,** examples will be discussed in connection with **FIG. 2A and 2B** below) configured to sense two or more different ECG leads at the augmented heart.

In the example of **FIG. 1** the cardiac augmentation device is configured to augment a human heart 61. However, the techniques of the present disclosure are not limited to application in human subjects. In other examples, the techniques of the present application can be deployed in an animal, in particular in other mammals than humans (for example, the techniques of the present disclosure can be used in cardiac augmentation techniques for primates, odd- or even-toed ungulates, rodents or other mammals). In general, the physiological conditions with respect to the electrical activity of the heart in these other animals and humans are sufficiently similar so that the techniques of the present disclosure can be applied to the particular subject with appropriate adaptations.

In the example of **FIG. 1****,** the actuator 2 is arranged within the pericardium 6 of the subject's heart 61. A connector 5 is provided to connect the actuator 2 with an external controller 30 (via another connector 91 arranged at a housing of the external controller 30).

The controller 30 is configured to determine one or more control parameters for the cardiac augmentation device using one or more of the two or more ECG leads and control operation of the actuator based on the determined one or more control parameters (these operations will be discussed in more detail below). In the example of **FIG. 1****,** all components of the controller 30 are arranged in the housing of the external controller 30 carried by the subject. In other examples, the actuator and/or the controller can be shaped and/or arranged differently.

For example, the controller can be included in an implantable device. In still other examples, the controller can be integrated with another device carried by the subject. In addition or alternatively, the controller can be connected to the actuator (and the sensor system) through a wireless connection (e.g., a short-range wireless connection).

The actuator 2 can have a large variety of different configurations. In one example, the actuator can include one or more (e.g., three or three or more) inflatable and deflatable pads. In this example, the controller 30 pressurizes and de-pressurizes the pads to effect augmentation of the heart 61. In other examples, the cardiac augmentation device can use a different actuator to apply pressure to a heart to augment a pumping function of the heart. For example, the device can include actuators based on electroactive materials or hydraulic actuators.

The controller can implement its functions by any combination of hardware and software. For example, the controller can include one or more general purpose processors and a memory storing appropriate instructions to carry out the operations described herein. In other examples, the controller can include dedicated hardware to carry out one or more of the operations described herein.

In addition or alternatively, the controller is not necessarily a single component but can also be distributed over a plurality of (spaced apart) components. For example, the controller can include a first implanted component and a second external component. The actual implementation of the controller is immaterial for the techniques of the present disclosure.

Turning to **FIG. 2A** and **FIG. 2B****,** an example sensor system of the cardiac augmentation device will be discussed in more detail. The sensor system includes a plurality of electrodes contained in a plurality of electrode elements 20a-20i.

In the example of **FIG. 2A** and **FIG. 2B****,** the electrode elements are epicardial electrode elements 20a-20i adapted to record the two or more different ECG leads. This means the electrode elements are attached to an epicardium of the heart.

In addition or alternatively, the electrode elements can include one or more electrode elements arranged in a different manner. For example, the sensor system can include one or more (in some cases only) externally attached electrode elements. In other examples, the sensor system can include electrode elements which are also used by other devices and/or part of other devices than the cardiac augmentation device. In one example, the cardiac augmentation devices of the present disclosure can use the electrode elements of a cardiac rhythm management device (e.g., electrode elements positioned inside the heart or vessels connected to the heart) to record the two or more ECG leads.

Returning to **FIG. 2A** and **FIG. 2B****,** the electrode elements 20a-20i are arranged in a predetermined spatial configuration (in other examples, the electrode elements can be arranged in different configurations at the heart). In the example of **FIG. 2A****,** three electrode elements 20a-20c are arranged about the heart. In the example of **FIG. 2A****,** six electrode elements 20h-20i are arranged at two different heights (measured from the apex of the heart) about the heart.

In other examples, the sensor system can include only two electrode elements, or a different number of electrode elements (e.g., four or more or five or more electrode elements).

Each electrode element 20a-20i can include one electrode, two electrodes (if an electrode element includes only one electrode, the two terms can be used interchangeably), or more than two electrodes (i.e., an electrode element is an structural assembly including the one or more electrodes). An "electrode" as used herein is a structure which is configured so that a single signal path leads from the structure to a circuit evaluating the signal measured by the structure. Thus, an electrode element having two electrodes is connected to a circuit evaluating the signals measured by the electrode elements via two signal paths, one for each electrode. Likewise, an electrode element having three or more electrodes is connected to a circuit evaluating the signals measured by the electrode elements via three or four signal paths, respectively. In general, two or more electrodes can be used to record one lead (as a lead cane encode a voltage difference between the two electrodes).

In one example, two electrodes of a single electrode element (e.g., of the electrode elements 20a-20i in **FIG. 2A** or **FIG. 2B****)** are configured to record a lead of the two or more leads of the sensing system as a voltage between the two electrodes. Along the same line, one or more leads of the two or more leads can be detected between the multiple electrodes of an electrode elements having more than two electrodes.

In some examples, one or more of the electrodes of an electrode element, or the electrode elements themselves can be spaced apart by less than 1 cm measured center-to center (e.g., less than 0.5 cm). For example, electrode elements can be epicardial electrode elements having at least two electrodes spaced apart less than 1 cm measured center-to center (e.g., less than 0.5 cm).

In addition or alternatively, the electrodes of different electrode elements (and its electrodes, respectively) can be used to record leads of the two or more different ECG leads. For example, electrodes of two electrode elements placed at different positions at or near a heart can be used to record a lead. In the example of **FIG. 2A****,** one lead could be recorded between a first electrode of (first) electrode element 20a and a second electrode of (second) electrode element 20b and a second lead between a third electrode of (third) electrode element 20c and the second electrode of the (second) electrode element 20b. Further leads can be recorded by combining other combinations of electrodes of different electrode elements.

In addition or alternatively, leads of the two or more different leads can be recorded by using the electrodes of different electrode elements and a (second) reference electrode (which can be an actual electrode or a reference potential).

In addition, as already mentioned above, leads of the two or more different leads can be calculated based on signals detected by the electrodes of different electrode elements. For instance, a lead between an electrode of electrode elements 20a and 20b could be calculated based on a first lead recorded between electrodes of electrode elements 20a and 20c and a second lead recorded between electrodes of electrode elements 20b and 20c.

Regardless of the layout and arrangement of the electrode elements and electrodes of the sensor system and regardless of if the leads are sensed directly or calculated, the sensor system is configured to record two or more different ECG leads at the augmented heart. For example, the sensor system can be configured to record three or more different ECG leads, or six or more different ECG leads, or nine or more different ECG leads.

In this manner, the augmentation control parameter detection process can be more reliable and robust, and can include deriving and using additional information compared to some determination processes including only a single lead. The details of the determination process will be explained below. However, before that different basic aspects of the pressure curves and electric activity characteristics of the heart will be shortly discussed in connection with **FIG. 3** to simplify understanding the subsequent sections.

### Dynamic pressure, volume and electric activity of the heart

**FIG. 3** shows example pressure, volume and ECG signals of left ventricle and adj acent vessels of a (healthy) heart. As can be seen, the pressure, volume and ECG signals are (substantially) periodic. For the cardiac augmentation, the pressure and volume situation in the ventricle are of primary concern. As can be seen in the upper curve, the pressure in the ventricle raises from a low (near zero) level after closure of the mitral valve. At a particular pressure level, the aortic valve opens (the period of time between closure of the mitral valve and opening of the aortic valve is called "isovolumic contraction" as the ventricle contracts without substantial change in pressure) and blood is ejected from the ventricle into the aorta. The blood volume in the ventricle drops accordingly during this ejection phase. At a later point in time, the aortic valve closes which means that no more blood can leave the ventricle. In the following isovolumic relaxation phase the ventricular pressure drops again towards the low (near zero) level. The mitral valve opens again which initiates the refilling process of the ventricle. This process repeats itself again and again. It will be discussed below that the actuators of a cardiac augmentation device shall be actuated at dedicated points in time of the cycle of ventricular ejection and refilling.

As indicated in **FIG. 3****,** the features of the ECG signals are temporally correlated with the above described periods of the ventricular pressure/volume cycle. This is not surprising as the ECG signal reflects the electric activity of the heart which leads to a contraction of the ventricles. Particularly, it can be seen that an R-wave of the ECG signal correlates with a start of the isovolumetric contraction phases when the ventricular pressure rises. On the other hand, an end of T-wave correlates with a point in time when the aortic valve closes and the ventricular pressure drops rapidly (i.e., the isovolumic relaxation phase).

It will be discussed subsequently in connection with **FIGS. 4A, 4B****, and** **5** how these correlations can be used in the cardiac augmentation techniques to determine control parameters for the cardiac augmentation device.

### Control parameters determined based on ECG leads

**FIG. 4A** depicts an example signal of an ECG lead 50a and corresponding pressure curves of an example cardiac augmentation device according to the present disclosure.

In particular, the ECG signal shows several desired augmentation time points, i.e., augmentation start and augmentation end points 40a, 40b.

The lower curve depicts again the left ventricular pressure and pressure curves of a pad of a cardiac augmentation device (e.g., a particular actuator) and an inflow valve of this pad. In many situations, it is desirable that the pressure applied by the actuators (e.g., a pad) follows the natural mechanical activity of the heart. At most, the cardiac augmentation device should apply pressure to the heart synchronously with the mechanical activity of the heart. In other words, it should be avoided (and might even be catastrophic) that the cardiac augmentation device falls out of sync with the natural activity of the heart.

In the example device of **FIG. 4A****,** the augmentation start point is a point in time when an inflow valve of the actuator opens. With a particular delay (e.g., caused by a length of tubing between the inflow valve and the pad) the pressure inside the pad opens which effects augmentation of the heart. Accordingly, an augmentation end point is a point in time where an outflow valve opens which leads to a rapid deflation of the pad. In cardiac augmentation devices with other actuators than pads the concrete components might be different. However, in these examples there will also be an augmentation start point at which application of pressure of the actuator starts and an augmentation end point at which the augmentation ends.

Coming back to the set of curves of **FIG. 4A****,** it can be seen that the augmentation start and end points correlate with features of the ECG signal.

In particular, the augmentation start point 40a correlates with an R-wave of the ECG lead 50a (e.g., the augmentation start point is selected to fall within a predetermined period of time after an R-wave). In general, determining an augmentation start point based on the one or more selected ECG leads can include identifying an R-wave.

The augmentation end point 40b corresponds with the end of a T-wave of the ECG lead 50a. In general, determining an augmentation end point based on the one or more selected ECG leads can include identifying a feature of a T-wave (e.g., an end of a T-wave). The augmentation end point can be a point in time in an isovolumetric relaxation phase of a ventricle.

The augmentation start point 40a and the augmentation end point 40b can be two control parameters of the cardiac augmentation device according to the present disclosure. Thus, the controller can determine the augmentation start point 40a and the augmentation end point 40b for the cardiac augmentation device using one or more of the two or more ECG leads. In other examples, the controller can determine only an augmentation start point 40a for the cardiac augmentation device using the one or more of the two or more ECG leads. In these examples, the augmentation end point 40b can be calculated or derived in a different manner than based on the two or more ECG leads. For instance, an augmentation end point can be calculated as a fixed or variable point in time after an augmentation start point 40a for the cardiac augmentation device determined using the one or more of the two or more ECG leads.

In addition or alternatively, other augmentation control parameters than an augmentation start point and/or an augmentation end point can be determined based on the one or more of the two or more ECG leads. For instance, the augmentation control parameters can include any parameter defining the interaction of the actuator of the heart. For instance, an augmentation control parameter can define a temporal and/or amplitude characteristic of the pressure the actuator applies to the heart (e.g., a pressure rise speed, a pressure drop speed, a complete pressure curve or parts thereof).

As can be seen in **FIG. 4A** and **FIG. 4B****,** the one or more control parameters (e.g., the augmentation start point, the augmentation end point, or both) can be determined for a single augmented contraction of the heart (i.e., a single heartbeat). In other examples, the one or more control parameters (e.g., the augmentation start point, the augmentation end point, or both) can be determined for multiple augmented contractions of the heart (e.g., a sequence of two or more augmented contractions or heartbeats).

In the example of **FIG. 4A** and **FIG. 4B****,** a single ECG lead 50a is shown to illustrate how an augmentation start point 40a and an augmentation end point 40b are determined. However, the techniques of the present invention include using two or more different ECG leads. This can happen in a plurality of different ways, as will be discussed in the following sections.

### Using two or more leads to determine the control parameters

**FIG. 4B** depicts example signals of a plurality of ECG leads 50b-50g according to the present disclosure. For example, the plurality of ECG leads 50b-50g can be recorded by using any one of the sensing systems described above in connection with **FIG. 2A** and **FIG. 2B****.**

As can be seen, the plurality of ECG leads 50b-50g are generally similar but also show quantitative and even qualitative variations. The position of the R-waves and the ends of the T-waves are highlighted in **FIG. 4B** (whereas the R-waves are labelled by triangles and the ends of the T-waves are labelled by squares). As discussed above, a detected R-wave 40c can be used to determine an augmentation start point while a detected end of a T-wave 40d can be used to determine an augmentation end point for the cardiac augmentation device.

In general, the controller of the cardiac augmentation device can be configured to dynamically select the one or more of the two or more different ECG leads 50b-50g to be used for determining one or more control parameters for the cardiac augmentation device.

In some example, the one or more control parameters are determined based on a single ECG lead of the two or more ECG leads. For example, the controller can select a first lead to determine the one or more augmentation control parameters for a first augmented heartbeat or a first series of more than one augmented heartbeats. In some examples, the controller can be configured to select a different lead of the two or more different ECG leads at different points in time. For instance, the controller can be configured to select a second lead to determine the one or more augmentation control parameters for a second augmented heartbeat or a second series of more than one augmented heartbeats following the first heartbeat / first series.

In **Fig. 4B****,** e.g., a first lead 50b can be selected for a first heartbeat, a second lead 50c can be selected for a second heartbeat and a third lead 50f can be selected for third heartbeat, and so on.

The change of the selected lead can happen after every heartbeat (when the controller determines that a different lead than the actual lead should be selected). In other situations, the same lead can be (or maintain) selected for multiple heartbeats. In still other situations, different leads can be selected for determining different augmentation control parameters for a single heartbeat (e.g., a first lead can be selected for determining the augmentation start point and a second, different lead can be selected for determining an augmentation end point of a single heartbeat).

In general, the controller applies one or more criteria to select the lead which is used to determine the augmentation control parameters. These criteria will be discussed next.

In some examples, selecting one or more of the two or more different ECG leads includes analyzing signals of the two or more different ECG leads 50b-50g. The analyzation step can include a plurality of different operations. Some example operations for the analyzation step will be discussed in the subsequent sections.

In some examples, the analyzing signals of the two or more different ECG leads can include determining a predefined characteristic of each of the two or more different ECG leads. The characteristic can be a position in time of a salient feature of the signal of the respective ECG lead. The salient feature can be a position in time of a T-wave, a position in time of an R-wave, a position in time of a Q-wave or a position in time of an S-wave, or a combination of two or more of these features. A position in time can be a position in time of an onset of one of these waves (e.g., an R-wave or a T-wave), a position in time of an extremum (i.e., a maximum or a minimum) of the wave, or a position of time of an end of the wave.

The analyzing step can alternatively or additionally include determining other features than a position in time of different waves of the signals of the ECG leads. For example, the salient feature can be a signal value or signal change exceeding a predetermined threshold (or exceeding a predetermined threshold for a predetermined duration of time).

However, particularly for determining an augmentation start point and/or an augmentation end point as a control parameter, a position in time of an R-wave and a position in time of an end of a T-wave can be a suitable salient feature of the ECG signals. As discussed above, these features in the ECG signals can correlate with the points in time suitable for starting and stopping applying pressure to the heart by the actuator of the cardiac augmentation device.

Coming back to the analyzing step discussed above, the analyzing operation can include comparing the signals of the two or more different ECG leads 50b-50g. For example, the analyzing step can include comparing the determined characteristic of the two or more different ECG leads and selecting the one or more of the two or more different ECG leads to be used based on a result of the comparison.

For example, as can be seen in **Fig. 4B****,** a position in time of an end of a T-wave varies between the different ECG leads 50a-50f for every heartbeat. In this case, a comparison of the characteristic of the two or more different ECG leads might yield that in one of the leads an end of the T-wave is detected earlier than in the other leads. In this case, the lead with the earlier detection point might not be selected for determining the augmentation control parameters. In other examples, the comparing step might allow identifying a lead for which the characteristic has a value between the values determined for other leads. In this example, this lead can be selected for determining the augmentation control parameters.

For instance, for determining an augmentation end point in the example of **FIG. 4B** including six leads, the third detected end of an T-wave can be used to determine the augmentation end point (i.e. the respective lead is selected).

It might be the case that a particular predefined characteristic cannot be determined for one heartbeat or for a sequence of heartbeats for one or more particular leads (e.g., due to transient or lasting effects on the particular lead). In this case, the particular lead is not selected for determining the augmentation control parameters.

In addition or alternatively, the analyzing operation of signals can include combining information retrieved from signals of the two or more different ECG leads.

For example, combining the information retrieved from signals of the two or more different ECG leads can include calculating one or more statistical parameters of an ensemble of signals of the two or more different ECG leads.

In addition or alternatively, combining the information retrieved from signals of the two or more different ECG leads can include determining a predefined characteristic of each of the two or more different ECG leads, calculating a detection parameter based on the determined predefined characteristic of each of the two or more different ECG leads and determining the one or more control parameters for the cardiac augmentation device using the detection parameter.

One example of this technique might involve constantly updating a detection parameter based on information retrieved from the two or more different ECG leads and determining an augmentation control parameter as soon as the detection parameter meets a predefined criterion (e.g., exceeds a predefined threshold). For example, the detection parameter might be increased or decreased as soon as a particular characteristic (e.g., an R-wave or an end of a T-wave) is detected in any one of the two or more different ECG leads. In addition, the detection parameter can be decreased or increased with time. As soon as the detection parameter exceeds a predetermined threshold, the one or more augmentation control parameters can be determined. In one illustrative example, the detection of an R-wave increases the detection parameter by 0.5. Moreover, the detection parameter is decreased by a predetermined amount per ms. As soon as the detection parameter exceeds a threshold of 0.8, an augmentation start point is determined. As can be seen, two detections of an R-wave in different leads in short succession can increase the detection parameter beyond the threshold and trigger determination of the augmentation start point. In this manner, it can be avoided that an augmentation is triggered by two R-waves spaced apart by a relatively long time interval.

In addition or alternatively, analyzing signals of the two or more different ECG leads can include determining a quality criterion for the signals of each of the ECG leads. In this case, ECG leads having a signal with a higher quality are preferred for use in determining the or more control parameters over ECG leads having a signal with a lower quality.

For instance, the quality criterion might be an indicator of a quantity of noise on the particular lead (e.g., a signal-to-noise ratio or another quantitative measure for noise). In addition or alternatively, the quality criterion might be an indicator of how reliable a predefined characteristic (e.g., or more of the characteristics discussed above) is determined for the particular lead. For example, it might be the case that for a certain lead the position in time of an R-wave and/or the position in time of an end of a T-wave could not be determined once or several times. In this case, the particular lead might be disregarded for a period of time for determining the augmentation control parameters.

In still other examples, determining a quality criterion can include evaluating a parameter of the signal of the lead (e.g., a baseline drift of the signal, an amplitude of the signal, a ratio of amplitudes of the signal such as a ratio of the amplitudes of the R-wave and the T-wave, and so on). In addition or alternatively, determining a quality criterion can include executing a plausibility test (e.g., a detected R-wave is followed by a detected end of a T-wave and so on). In still other examples, determining a quality criterion can include evaluating an auxiliary parameter (e.g., determining that an analogue-digital-converter employed in recording the lead saturates). As in the examples above, a lead can be disregarded temporarily or permanently depending on the outcome of the quality criterion determination.

In addition or alternatively, analyzing signals of the two or more different ECG leads can include analyzing statistical data of the signals of each of the ECG leads. For example, the use of statistical data might include determining an average (or other statistical measure) of a predefined characteristic (e.g., or more of the characteristics discussed above) for past heartbeats. This information can then be used by the controller to select a lead for the determination of augmentation control parameters. For instance, a lead for which the predefined characteristic has fallen within a predetermined environment of an average predefined characteristic of all leads can be selected. On the other hand, a lead that has produced outliers compared to the other leads can be disregarded.

In addition or alternatively, analyzing signals of the two or more different ECG leads can include analyzing historical data of the signals of each of the ECG leads. This might include storing and analyzing the signals (or information retrieved from the signals) for a predetermined period of time. The historical data can include the measurement signals of the leads themselves, or any derived parameters discussed above (e.g., determined characteristics of the leads, statistical data or quality criteria for the leads).

In still other examples, analyzing the signals can include modifying one or more analysis results of the past. For instance, a detected salient feature of a lead can be ignored based on one or more criteria (e.g., after a predetermined amount of time has lapsed). In one example, an isolated R-wave can be detected which is ignored if no further R-wave is detected within a predetermined period of time after detection of the first R-wave.

In still other examples, analyzing the signals can include using additional information of one or more additional data sources. In one example, one or more physiological parameters of a patient can be taken into account (e.g., a heart rate, a respiration rate or other physiological parameters). In still other examples, an external parameter can be taken into account (e.g., a time of the day). These additional parameters can also be used in the process of determining the augmentation control parameters.

As described in the previous sections, the signals of the two or more leads can be analyzed in various ways to select a suitable lead (or multiple suitable leads) based on whose signals the augmentation control parameters can be determined. In many cases, this determination procedure is carried out periodically or continuously (e.g., for every heartbeat or even multiple times within one heartbeat). However, in some examples a particular selection is used permanently (e.g., after a test phase in which a suitable lead or a group of suitable leads is determined). In any case, the techniques of the present disclosure use information of two or more leads of an ECG of the augmented heart. This can have different advantages in terms of robustness and accuracy of the determination technique in some examples.

Additional details of the determination of the augmentation control parameters will be discussed next.

### Determination of augmentation control parameters

In general, the controller of the techniques of the present disclosure can determine the one or more control parameters (e.g. the augmentation start point ) in real time. For example, the one or more control parameters can be determined with a latency of below 30 ms (e.g., below 25 ms) in some examples. In this manner, information retrieved from the signals of the two or more leads about a current heartbeat can be used to control the augmentation for a contraction cycle of this very heartbeat. In other examples, the controller can use information retrieved for previous heartbeats to control the augmentation of a current contraction cycle (even though this might be not desirable in some situations).

In general, the step of determining the augmentation control parameters can include a number of additional steps (e.g., signal processing steps) which are not described herein. For example, the points in time discussed above detected in the signals of the one or more leads can be translated into a timing sequence for controlling one or more components of the cardiac augmentation device effecting the operation of the device (e.g., the actuator applying pressure to the heart).

This processing can include many different steps. For example, it has been discussed above that the position in time of an R-wave or the end of a T-wave can be used to determine an augmentation start point and an augmentation end point, respectively.

This can mean that based on the determined point in time (e.g., of an end of a T-wave), one or more augmentation control parameters are derived according to a predetermined set of instructions. The nature of the instructions can vary depending on, e.g., the type of the cardiac augmentation device, the application situation, or other factors. Accordingly, the exact nature of the processed augmentation control parameters might vary.

For instance, an augmentation control parameter for an augmentation end point might be a command to open one or more valves to effect a deflation of a pad of the cardiac augmentation device at a predetermined point in time. When using different hardware in the cardiac augmentation device, the nature of the processed augmentation control parameters might change accordingly. In other examples, the processing of the augmentation control parameters might include taking into account latencies in the cardiac augmentation device (e.g., a latency between opening a valve for deflating a pad an actual onset of a pressure drop).

In some examples above, it has been shown that a cardiac augmentation control parameter is determined based on information from a signal of a single (selected) lead of the two or more leads. In other examples, the one or more control parameters are determined based on multiple ECG leads of the two or more ECG leads.

For instance, determining the one or more control parameters includes combining information of multiple ECG leads of the two or more different ECG leads. In some examples, combining information of multiple ECG leads includes calculating a weighted average of the information of multiple ECG leads. For instance, the controller might use an average of a point in time of a characteristic of the leads (e.g., a position in time of an R-wave or end of a T-wave) to determine an augmentation control parameter.

The controller of the cardiac augmentation device of the present disclosure can be configured to adapt a process of determining the one or more control parameters based on a number of available ECG leads. For instance, a first sensor system might be configured to record six leads while a second sensor system might be configured to record twelve leads. The controller can be adapted to take into account a variation of recordable leads. In addition or alternatively, a number of available leads might vary due to transient or permanent malfunctions of the electrode elements or other hardware of the cardiac augmentation device. For instance, an epicardia electrode element might have insufficient contact with the heart to record a lead. The controller can also be configured to take into account these situations.

As discussed above, **FIG. 4B** depicts example signals for a plurality of ECG leads according to the present disclosure. In the example of **FIG. 4B****,** six leads 50b-50g are recorded (which might be sensed directly or calculated). R-waves (e.g., R-wave 40c) and ends of T-waves (e.g., end of T-wave 40d) are also indicated in **FIG. 4B** (as triangles and squares, respectively). As discussed above, the R-waves can be used to determine an augmentation start point and the ends of the T-waves can be used to determine an augmentation end point. It can be seen in **FIG. 4B** that the points in time of the detected features vary for different leads. This shows that having two or more leads for determining the augmentation control parameters can be helpful to achieve a robust and accurate determination of the parameters.

In the preceding sections, the determination of augmentation control parameters has been discussed in some detail mainly based on the examples of an augmentation start point and an augmentation end point. However, the techniques of the present disclosure can also involve determining other parameters. In addition, recording two or more ECG leads in a sensor system of a cardiac augmentation also facilitates implementing other functions. This will be explained in more detail in the following sections.

### Additional features

In some examples, the controller is configured to detect an indication of one or more pathological or non-pathological conditions of the heart in a signal of the two or more ECG leads. The one or more pathological or non-pathological conditions can include one or more of an arrhythmia, an ischemia, a heart attack or an inflammatory condition. For instance, arrhythmias can include tachycardia, bradycardias, heart fluttering (atrial or ventricular) or different conduction blocks of the heart. Moreover, arrhythmias can include extra systoles, couplets, bigemini, trigemini salvos or other irregular pattern in the electrical activity of the heart.

In some examples, detecting the one or more pathological or non-pathological conditions of the heart includes comparing signals of the two or more ECG leads. For instance, the controller can be configured to compare positions in time of predefined characteristics of the signals of the different ECG leads (e.g., determine latencies). In the example of detecting the R-wave (and/or an end of a T-wave), latencies between the detected positions in time can be used to derive that one or more pathological or non-pathological conditions are present.

In some examples, the controller can be configured to adapt a process of determining the one or more control parameters based on the detected one or more pathological or non-pathological conditions of the heart. For instance, the controller is configured to identify the occurrence of an arrhythmia in the signals of the two or more ECG leads and use this information when selecting the one or more of the two or more different ECG leads to be used for determining one or more control parameters. For instance, when detecting a block in the heart or an ischemia, signals of electrode elements attached to the affected parts of the heart can be disregarded.

In addition or alternatively, upon identifying a particular pathological or non-pathological condition of the heart, the controller can adapt the determination process of the augmentation control parameters.

This can include one or more of the following steps.

In some examples, the controller can be configured to determine information regarding an asynchronicity of activity of the heart based on the two or more ECG leads. Then, determining the one or more control parameters may include using the determined information regarding an asynchronicity of activity of the heart. For instance, a block can lead to higher latencies between the signals of the different ECG leads. In other examples, a fluttering or fibrillation can render some ECG leads useless. The determination process of the augmentation control parameters can be adapted accordingly.

In other examples, the controller can identify an asynchronous contraction of the two ventricles of the heart. In this case, the controller is configured to resynchronize the heart by applying respective pressure by the actuator (e.g., inflating multiple pads of a cardiac augmentation device including inflatable pads in a predetermined sequence).

In addition or alternatively, the controller can be configured to determine amplitudes of an excitation of the heart in the two or more ECG leads, and adjust pressure applied to the heart based on the determined amplitudes.

In addition or alternatively, the controller can be configured to dynamically determine a 3-dimensional polarization vector of the heart based on multiple ECG leads. In these examples, determining the one or more control parameters includes using the determined 3-dimensional polarization vector of the heart.

In addition or alternatively, the controller is configured to determine a respiratory frequency based on the two or more different ECG leads and using the determined respiratory frequency to determine the one or more control parameters.

### Machine learning

The processes for selecting one or more of the two or more leads and determining the one or more augmentation control parameters have been described in some detail in the preceding sections. As discussed, these processes can happen according to a variety of different algorithms.

The determination algorithm can be fixed or dynamic. For instance, the determination algorithm can be updated after deploying the cardiac augmentation device. In addition or alternative, the determination algorithm can be adaptable to adapt itself to a particular deployment situation (in some examples automatically or in supervised training phase).

In one example, determining one or more control parameters for the cardiac augmentation device using one or more of the two or more ECG leads includes using a module trained by machine learning. In addition or alternatively, the controller can be configured to detect an indication of one or more pathological or non-pathological conditions of the heart using a module trained by machine learning. The machine learning process can happen once prior to the deployment of the cardiac augmentation device, or intermittently or continuously during deployment of the cardiac augmentation device.

### Calibration process

In the previous sections it has been described how a controller of a cardiac augmentation device can determine augmentation parameters based on two or more ECG leads. In some examples, the cardiac augmentation device can be calibrated to further improve the determination process. For instance, a calibration process can include obtaining information regarding anatomical and/or physiological conditions of a patient. In one example, the information regarding anatomical and/or physiological conditions of a patient can include data obtained by one or more diagnostic procedures (e.g., an examination of the patient via catheter, via an imaging technique of a via a physical examination). The information regarding anatomical and/or physiological conditions of a patient can be used to establish a relationship between electrical and mechanical conditions in the heart of the specific patient. For example, the calibration process can include determining a latency between an onset or end of a mechanical systole of the patient's heart and salient features of the signals of the two or more ECG leads.

Thus, during the calibration process, the controller can adapt one or more parameters of the augmentation control parameter generation process based on the obtained information regarding anatomical and/or physiological conditions of the patient.

### Computer program product and method

In the preceding sections, different aspects of a cardiac augmentation device have been described. However, the present disclosure also relates to corresponding methods for determining control parameters for a cardiac augmentation device and computer program products including instructions stored thereon which when executed by a controller of a cardiac augmentation device prompt the cardiac augmentation device to carry out the operation described in the present disclosure.

**FIG. 5** includes a flow diagram of an example method according to the present disclosure.

The method includes determining control parameters for a cardiac augmentation device, the cardiac augmentation device including an actuator configured to apply pressure to a heart to augment a pumping function of the heart and includes detecting two or more different ECG leads at an augmented heart and determining one or more control parameters for the cardiac augmentation device using one or more of the two or more ECG leads 103.

In addition, the method can further comprise dynamically selecting the one or more of the two or more different ECG leads to be used for determining one or more control parameters for the cardiac augmentation device 102 and, optionally, controlling operation of an actuator of the cardiac augmentation device based on the determined one or more control parameters 104.

Additionally, the method can include any of the other methods steps (particularly steps performed by the controller of the cardiac augmentation device) described in the present disclosure.

Likewise, the present disclosure relates to a computer program products including instructions stored thereon which when executed by a controller of a cardiac augmentation device prompt the cardiac augmentation device to detect two or more different ECG leads at an augmented heart, determine one or more control parameters for the cardiac augmentation device using one or more of the two or more ECG leads of the cardiac augmentation device and control operation of an actuator of the cardiac augmentation device based on the determined one or more control parameters 104.

The computer program product can further include instructions stored thereon which when executed by the controller of a cardiac augmentation device prompt the cardiac augmentation device to dynamically select the one or more of the two or more different ECG leads to be used for determining one or more control parameters for the cardiac augmentation device 102.

Additionally, the computer program product can include instructions to carry out any of the other methods steps (particularly steps performed by the controller of the cardiac augmentation device) described in the present disclosure.

## Claims

1. A cardiac augmentation device (10), including:
an actuator (2) configured to apply pressure to a heart (61) to augment a pumping function of the heart;
a sensor system configured to record two or more different ECG leads (50a-50g) at the augmented heart;
a controller (30) configured to:
determine one or more control parameters (40a-40d) for the cardiac augmentation device (10) using one or more of the two or more ECG leads; and
control operation of the actuator based on the determined one or more control parameters.

2. The cardiac augmentation device of claim 1, wherein the controller is further configured to:
dynamically select the one or more of the two or more different ECG leads to be used for determining one or more control parameters for the cardiac augmentation device.

3. The cardiac augmentation device of claim 1 or claim 2, wherein the sensor system includes two or more epicardial electrode elements (20a-20i) adapted to record the two or more different ECG leads.

4. The cardiac augmentation device of any one of the preceding claims 1 to 3, wherein the one or more control parameters are determined based on multiple ECG leads of the two or more ECG leads.

5. The cardiac augmentation device of any one of the preceding claims 2 to 4, wherein selecting one or more of the two or more different ECG leads includes analyzing signals of the two or more different ECG leads.

6. The cardiac augmentation device of claim 5, wherein analyzing signals of the two or more different ECG leads includes:
determining a predefined characteristic of each of the two or more different ECG leads;
calculating a detection parameter based on the determined predefined characteristic of each of the two or more different ECG leads; and
determining the one or more control parameters for the cardiac augmentation device using the detection parameter.

7. The cardiac augmentation device of any one of the preceding claims 2 to 6, wherein the controller is configured to identify the occurrence of an arrhythmic condition in the signals of the two or more ECG leads and use this information when selecting the one or more of the two or more different ECG leads to be used for determining one or more control parameters.

8. The cardiac augmentation device of any one of the preceding claims, wherein the controller is configured to dynamically determine a 3-dimensional polarization vector of the heart based on the two or more ECG leads.

9. The cardiac augmentation device of any of the preceding claims, wherein determining one or more control parameters for the cardiac augmentation device using one or more of the two or more ECG leads includes using a module trained by machine learning.

10. A computer program product including instructions stored thereon which when executed by a controller of a cardiac augmentation device prompt the cardiac augmentation device to:
record two or more different ECG leads at an augmented heart (101);
determine one or more control parameters for the cardiac augmentation device using one or more of the two or more ECG leads of the cardiac augmentation device (103); and
control operation of an actuator of the cardiac augmentation device based on the determined one or more control parameters (104).

## Patentansprüche

1. Herzaugmentationsvorrichtung (10), umfassend:
einen Aktor (2), der so konfiguriert ist, dass er Druck auf ein Herz (61) ausübt, um eine Pumpfunktion des Herzens zu steigern;
ein Sensorsystem, das so konfiguriert ist, dass es zwei oder mehr verschiedene EKG-Ableitungen (50a-50g) am augmentierten Herzen aufzeichnet;
eine Steuereinheit (30), die konfiguriert ist zum:
Bestimmen eines oder mehrerer Steuerparameter (40a-40d) für die Herzaugmentationsvorrichtung (10) unter Verwendung einer oder mehrerer der zwei oder mehr EKG-Ableitungen; und
Steuern des Betriebs des Aktors basierend auf dem einen oder den mehreren bestimmten Steuerparametern.

2. Herzaugmentationsvorrichtung nach Anspruch 1, wobei die Steuerschaltung ferner konfiguriert ist zum:
dynamischen Auswählen der einen oder der mehreren der zwei oder mehr verschiedenen EKG-Ableitungen, die zum Bestimmen eines oder mehrerer Steuerparameter für die Herzaugmentationsvorrichtung verwendet werden sollen.

3. Herzaugmentationsvorrichtung nach Anspruch 1 oder 2, wobei das Sensorsystem zwei oder mehr epikardiale Elektrodenelemente (20a-20i) umfasst, die zum Aufzeichnen der zwei oder mehr verschiedenen EKG-Ableitungen ausgelegt sind.

4. Herzaugmentationsvorrichtung nach einem der vorhergehenden Ansprüche 1 bis 3, wobei der eine oder die mehreren Steuerparameter basierend auf mehreren EKG-Ableitungen der zwei oder mehr EKG-Ableitungen bestimmt werden.

5. Herzaugmentationsvorrichtung nach einem der vorhergehenden Ansprüche 2 bis 4, wobei das Auswählen einer oder mehrerer der zwei oder mehr verschiedenen EKG-Ableitungen ein Analysieren von Signalen der zwei oder mehr verschiedenen EKG-Ableitungen umfasst.

6. Herzaugmentationsvorrichtung nach Anspruch 5, wobei das Analysieren von Signalen der zwei oder mehr verschiedenen AKG-Ableitungen umfasst:
Bestimmen einer vordefinierten Charakteristik jeder der zwei oder mehr verschiedenen EKG-Ableitungen;
Berechnen eines Detektionsparameters basierend auf der bestimmten vordefinierten Charakteristik jeder der zwei oder mehr verschiedenen EKG-Ableitungen; und
Bestimmen des einen oder der mehreren Steuerparameter für die Herzaugmentationsvorrichtung unter Verwendung des Detektionsparameters.

7. Herzaugmentationsvorrichtung nach einem der vorhergehenden Ansprüche 2 bis 6, wobei die Steuerung so konfiguriert ist, dass sie das Auftreten eines arrhythmischen Zustands in den Signalen der zwei oder mehr EKG-Ableitungen identifiziert und diese Information beim Auswählen der einen oder der mehreren der zwei oder mehr verschiedenen EKG-Ableitungen verwendet, die zum Bestimmen eines oder mehrerer Steuerparameter verwendet werden sollen.

8. Herzaugmentationsvorrichtung nach einem der vorhergehenden Ansprüche, wobei die Steuerung so konfiguriert ist, dass sie einen 3-dimensionalen Polarisationsvektor des Herzens basierend auf den zwei oder mehr EKG-Ableitungen dynamisch bestimmt.

9. Herzaugmentationsvorrichtung nach einem der vorhergehenden Ansprüche, wobei das Bestimmen eines oder mehrerer Steuerparameter für die Herzaugmentationsvorrichtung unter Verwendung einer oder mehrerer der zwei oder mehr EKG-Ableitungen ein Verwenden eines durch maschinelles Lernen trainierten Moduls umfasst.

10. Computerprogrammprodukt, umfassend darauf gespeicherte Anweisungen, die bei Ausführung durch eine Steuerung einer Herzaugmentationsvorrichtung die Herzaugmentationsvorrichtung veranlassen zum:
Aufzeichnen zweier oder mehrerer verschiedener EKG-Ableitungen an einem augmentierten Herzen (101);
Bestimmen eines oder mehrerer Steuerparameter für die Herzaugmentationsvorrichtung unter Verwendung einer oder mehrerer der zwei oder mehr EKG-Ableitungen der Herzaugmentationsvorrichtung (103); und
Steuern des Betriebs eines Aktors der Herzaugmentationsvorrichtung basierend auf dem einen oder den mehreren bestimmten Steuerparametern (104).

## Revendications

1. Dispositif d'augmentation cardiaque (10), comprenant :
un actionneur (2) configuré pour appliquer une pression sur un coeur (61) afin d'augmenter la fonction de pompage du coeur ;
un système de capteur configuré pour enregistrer au moins deux dérivations ECG différentes (50a-50g) au niveau du coeur augmenté ;
un contrôleur (30) configuré pour :
déterminer un ou plusieurs paramètres de commande (40a-40d) pour le dispositif d'augmentation cardiaque (10) en utilisant une ou plusieurs des deux dérivations ECG ou davantage ; et
commander le fonctionnement de l'actionneur sur la base d'un ou plusieurs paramètres de commande déterminés.

2. Dispositif d'augmentation cardiaque selon la revendication 1, dans lequel le contrôleur est en outre configuré pour :
sélectionner dynamiquement les une ou plusieurs dérivations des deux, ou davantage, dérivations ECG différentes à utiliser pour déterminer un ou plusieurs paramètres de commande pour le dispositif d'augmentation cardiaque.

3. Dispositif d'augmentation cardiaque selon la revendication 1 ou la revendication 2, dans lequel le système de capteur comprend deux, ou davantage, éléments d'électrodes épicardiques (20a-20i) adaptés pour enregistrer les deux, ou davantage, dérivations ECG différentes.

4. Dispositif d'augmentation cardiaque selon l'une quelconque des revendications précédentes 1 à 3, dans lequel les un ou plusieurs paramètres de commande sont déterminés sur la base de plusieurs dérivations ECG parmi les deux, ou davantage, dérivations ECG.

5. Dispositif d'augmentation cardiaque selon l'une quelconque des revendications précédentes 2 à 4, dans lequel la sélection d'une ou plusieurs des deux, ou davantage, dérivations ECG différentes comprend l'analyse des signaux des deux, ou davantage, dérivations ECG différentes.

6. Dispositif d'augmentation cardiaque selon la revendication 5, dans lequel l'analyse des signaux des deux, ou davantage, dérivations ECG différentes comprend les étapes suivantes :
déterminer une caractéristique prédéfinie de chacune des deux, ou davantage, dérivations ECG différentes ;
calculer un paramètre de détection basé sur la caractéristique prédéfinie déterminée de chacune des deux, ou davantage, dérivations ECG différentes ; et
déterminer un ou plusieurs paramètres de commande pour le dispositif d'augmentation cardiaque à l'aide du paramètre de détection.

7. Dispositif d'augmentation cardiaque selon l'une quelconque des revendications précédentes 2 à 6, dans lequel le contrôleur est configuré pour identifier l'apparition d'un état d'arythmie dans les signaux des deux, ou davantage, dérivations ECG et utiliser cette information lors de la sélection de l'une ou plusieurs des deux, ou davantage, dérivations ECG différentes à utiliser pour déterminer un ou plusieurs paramètres de commande.

8. Dispositif d'augmentation cardiaque selon l'une quelconque des revendications précédentes, dans lequel le contrôleur est configuré pour déterminer dynamiquement un vecteur de polarisation tridimensionnel du coeur sur la base des deux, ou davantage, dérivations ECG.

9. Dispositif d'augmentation cardiaque selon l'une quelconque des revendications précédentes, dans lequel la détermination d'un ou plusieurs paramètres de commande pour le dispositif d'augmentation cardiaque à l'aide d'une ou plusieurs des deux, ou davantage, dérivations ECG comprend l'utilisation d'un module entraîné par apprentissage automatique.

10. Produit programme d'ordinateur comprenant des instructions stockées sur celui-ci qui, lorsqu'elles sont exécutées par un contrôleur d'un dispositif d'augmentation cardiaque, invitent le dispositif d'augmentation cardiaque à :
enregistrer deux, ou davantage, dérivations ECG différentes au niveau d'un coeur augmenté (101) ;
déterminer un ou plusieurs paramètres de commande pour le dispositif d'augmentation cardiaque en utilisant une ou plusieurs des deux, ou davantage, dérivations ECG du dispositif d'augmentation cardiaque (103) ; et
commander le fonctionnement d'un actionneur du dispositif d'augmentation cardiaque sur la base d'un ou de plusieurs paramètres de commande déterminés (104).
